# EUROPEAN PATENT SPECIFICATION

(11) **EP 1 677 100 B1**
(45) Date of publication and mention of the grant of the patent: **31.03.2010**
(21) Application number: 03751659.8
(22) Date of filing: 15.09.2003
(51) Int. Cl.: G01N 25/02

(54) **EQUILIBRIUM BOMB FOR STUDYING PHASE BEHAVIOUR OF HYDROCARBONS**
GLEICHGEWICHTSKOLBEN ZUR UNTERSUCHUNG DES PHASENVERHALTENS VON KOHLENWASSERSTOFFEN
CELLULE D'EQUILIBRE PERMETTANT D'ETUDIER LE COMPORTEMENT DE PHASE D'HYDROCARBURES

(30) Priority: 27.11.2002 RU 2002131993
(43) Date of publication of application: 05.07.2006
(73) Proprietor: Gazprom dobycha Orenburg, Orenburg 460021 (RU)
(72) Inventor: KOUVANDYKOV, Ilis Sharifovich, Orenburg, 460008 (RU); GAFAROV, Nail Anatolievich, Orenburg, 460008 (RU)
(74) Representative: Bucher, Ralf Christian
(86) International application number: PCT/RU2003/000403
(87) International publication number: WO 2004/048952

(56) References cited:
- SU-A1- 1 679 337
- SU-A1- 1 770 819
- SU-A3- 1 808 127
- US-A- 2 662 393
- US-A- 4 804 274
- US-A- 5 758 968
- VELIKOVSKY ET AL.: "STUDIES OF GAS CONDENSATE DEPOSITS" VNINGAZ, vol. 17/25, pages 265-269, Moscow, 1962

## Description

### FIELD OF THE INVENTION

The present invention relates to devices intended to study a phase behavior of hydrocarbons, and may be used in oil and gas industry for the research purposes when establishing the main parameters of depth and recombinant samples of stratified oils and gas-condensate systems brought to thermo-baric modes of their occurrence.

### BACKGROUND OF THE INVENTION

There has been known an equilibrium bomb for studying a phase state of a gas-condensate system PVT-7, comprising a cylinder with two pistons. Each piston has a rod with a measuring device that allows the fixation of the piston position in the bomb to the nearest 0.1 mm. In the middle of the lower part of the bomb, there are sight glasses to enable measurement, according to a liquid level, of the bomb's volume by setting the bomb at an angle of 45° and by taking a reading of a lower rod, in virtue of a specific pre-calibration carried out. In the upper part, there is a gas inlet-outlet valve, whilst in the lower one - a liquid inlet-outlet valve. Before the trials begin, the pistons are butt-jointed, and a space there-between is evacuated, followed by filling up the bomb with the gas and condensate. Following sampling, the bomb is preset with a given thermo-baric modes, followed by the intense swinging thereof. Upon establishing the equilibrium during 2 - 2.5 hours, a liquid phase volume is determined by bringing a liquid level to the middle of the said sight glass having a graduation line. Thereupon, the lower piston is brought to the level of the middle of the sight glass, and a liquid phase is evacuated under constant pressure. Following the liquid phase evacuation, a gaseous phase is evacuated [Studying Gas-Condensed Deposits. Edited by A.S. Velikovsky et al., VNIIgas Collection, Issue 17/25, M., Gostoptekhizdat, 1962, pp. 108-113].

However, the design of the known equilibrium bomb prevents the volume of a liquid phase precipitated from being measured to an accuracy that is necessary to study phase equilibriums.

There has also been known an equilibrium bomb for studying reservoir oils, comprising a cylinder with two pistons, one of which having its rod hollow. Inside the rod, there is disposed a bar whose inner end is extended with a perforated cylinder actuated with an electromagnet by means of which the admixture of samples is carried out. At each pressure stage, the sample is thoroughly mixed and kept up to the complete pressure stabilization. When a saturation pressure is achieved, a more resilient gaseous phase is formed, and a volume-to-pressure unit increment augments noticeably. On the plotted graph of the volume against pressure increment, a point of inflection corresponds to a pressure of oil saturation with gas [A.I. Khaznaferov. Studying Stratified Oils. Edited by V.N. Mamuny, M., Nedra, 1987, pp. 61-64].

The drawback to the known bomb resides in that there is no way to study the recently discovered "volatile" high gas-content oils, due to the absence on their PV-isotherm of a characteristic fracture corresponding to a saturation pressure.

The closest prior art with respect to the present invention resides in an equilibrium bomb for studying phase equilibriums of hydrocarbon systems at low temperatures [Studying Gas-Condensed Deposits. Edited by A.S. Velikovsky et al., VNIIgas Collection, Issue 17/25, M., Gostoptekhizdat, 1962, pp. 265-269]. The bomb comprises a body closed on its top by a cylinder. In the upper thickened part of the bomb, there is a through viewing port closed by sighting telescopes with strong triple lenses. Inside the body, there is a device for measuring the content of a liquid within the bomb, said device comprising a movable rule with graduation lines applied thereon and reflectors arranged in front of the lenses. At the bottom part of the rule, two mirrors are fixed at an angle of 45° with respect to the horizon. The upper end of the rule is fastened in a cylindrical iron core disposed inside the cylinder on which an electromagnet is mounted while drawing in said core. At the bottom part of the bomb body there is disposed an opening with a pipe union through which a pre-cooled vapor-liquid mixture enters the bomb. An opening with a pipe union arranged in the upper part of the bomb cylinder serve to release the vapor phase from the bomb, whereas being disposed in the side part they serve to afford sampling of the liquid.

The equilibrium bomb is filled with components of the system to be studied.
A required pressure is set by means of a measuring press. Then a mixture to be studied is stirred by means of a circulating pump until the equilibrium is established. A lighter arranged in front of one of the viewing ports produces a light beam which, while passing inside the bomb, is reflected from the first reflector downwards and precipitates on one of the rule mirrors, then on the other mirror and further upwards on the second reflector, where it may be fixed visually through the second viewing port. To measure the precipitated hydrocarbon liquid, the circulating pump stirring the vapor phase is turned off, the electromagnet is switched on and moved together with the drawn in iron core until bottom mirrors fastened on the rule touch to a liquid level, which may be observed through the viewing port. As a result, there are obtained data about the change in ratios of volumes of coexisting phases under different thermo-dynamic conditions.

The drawbacks to the known equilibrium bomb reside in low precision measurements of a precipitated liquid phase volume, especially with a low potential content of liquid hydrocarbons in the gas-condensate system and minor volumes of the liquid phase to be precipitated when the pressure is lowered, as well as impossibility to determine visually the pressure of saturating the stratified oil samples, since there is no possibility of a direct passage of the light beam from one port to the other. A double distance between upper reflectors and bottom mirrors fastened on the measuring rule results in a complete extinction of the luminous flux by dark oil.

### OBJECTS OF THE INVENTION

It is an object of the present invention to provide a multifunctional bomb for studying a phase behavior of stratified hydrocarbon systems and to increase precision of measurements of micro volumes of a liquid phase precipitated there from when lowering the pressure.

### SUMMARY OF THE INVENTION

This object is achieved by means of an equilibrium bomb as defined in claim 1.

A radial slot of the bottom reflector can be arranged perpendicular to the partition which is fixed with the aid of two grooves on the interior surface of the container in a perpendicular direction to the axis of the viewing ports.

The technical effect to be achieved at the expense of the availability of the dividing piston which is attached, with a fixed gap, to a bottom reflector, consists in that, apart from the known dividing function, there is also performed an additional function - to provide a means for the upright displacement of the reflectors in order to arrange them at the preset levels relative to the viewing ports, including at a level of a fixed gap between the bottom reflector and the piston, in order to enable a direct passage of the luminous flux from one port to another.

The technical effect to be achieved at the expense of embodying the reflectors in the form of cylinders provided with reflecting surfaces at an angle of 45° and arranged in the container with a narrow gap with respect to its interior wall, consists in the provision of an efficient stirring of the medium to be studied, not using a special stirrer, but by forcing thereof over narrow gaps when the piston with reflectors are forced over by means of a measuring press communicating with a space below the piston of the container.

The technical effect to be achieved at the expense of providing a cylindrical base of the bottom reflector with a radial slot consists in the provision of visual determination of a phase boundary of those micro volumes of the precipitated hydrocarbon condensate which are specially moved into a narrow gap between the bottom reflector and a container interior wall, resulting in a considerable increase in precision measurements of volumes of a precipitated liquid phase of the medium to be studied.

The availability of a rectangular partition with an opening at a level of the top reflector, said partition being fixed with the aid of two grooves on the interior surface of the container in a perpendicular direction to the axis of the viewing ports, makes it possible to observe through the viewing port only a luminous flux moving from the reflectors and thereby to eliminate an extraneous luminous background when recording a darkening point (dew point) of the gas-condensate system to be studied.

A bypass tube connecting, through a stop valve, spaces above and below the piston to each other, provides for the possibility to counterbalance, if need be, the pressure of a working agent below the dividing piston with the pressure of a reservoir fluid to be studied above the piston, and to fix a measuring system at a certain level with respect to the axis of the viewing ports at each stage of lowering the pressure in the equilibrium bomb.

Thus, the distinctive features of the claimed equilibrium bomb allow for determining a saturation pressure (bubble point) of reservoir oils, a pressure of starting condensation (dew point) of gas-condensate systems as well as an increase in precision measurements of micro volumes of a precipitated liquid phase, thereby enabling functionalities of the bomb when preserving its serviceability.

In the accessible sources of patent and other scientific and technological information, there have been revealed no data about technical solutions containing features corresponding to the distinctive features of the present invention and providing a similar technical effect. This makes it possible to draw a conclusion that the claimed technical solution is in conformity with the criteria of "novelty" and "inventive step".

### BRIEF DESCRIPTION OF THE DRAWINGS

In the drawings:

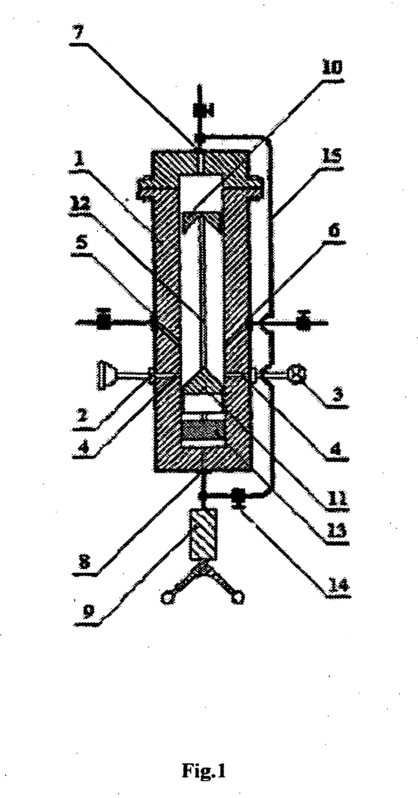

FIG. 1 illustrates an equilibrium bomb in accordance with the present invention;

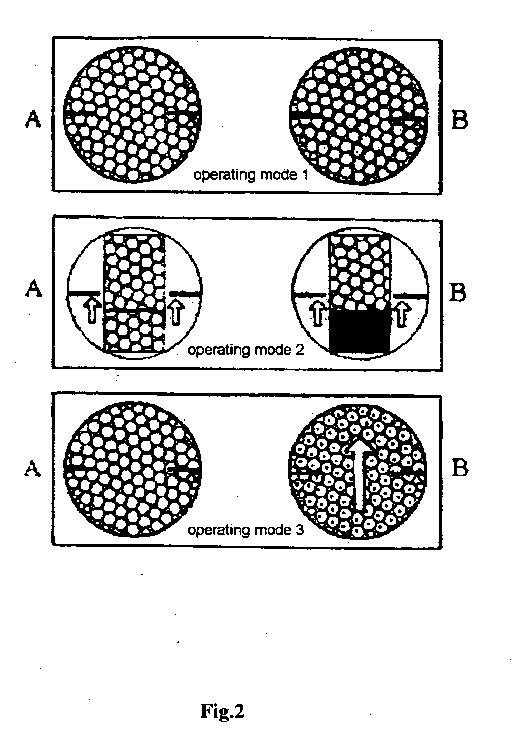

FIG. 2 illustrates images which are necessery and sufficiently proof to be recognized as to with the bomb's help, when studying a phase behavior of oil and gas-condensate systems in three operating modes according to the present invention.

### DETAILED DESCRIPTION OF THE INVENTION

An equilibrium bomb comprises a temperature-controlled high-pressure cylindrical container 1 with a cover, said container being provided on its lateral wall with viewing ports 2 at a level of a light source 3 and optical fiber bundles 4 embedded in drilled holes, and openings 5 and 6 with pipe unions to connect with temperature-controlled recombination wells or a thief tube. On the container ends, there are made openings 7 and 8 with pipe unions to connect a container cavity by means of steel capillary tubes respectively with a standard manometer and a measuring press 9. Inside the container, there are disposed a top 10 and a bottom 11 reflectors being rigidly connected to each other by means of a rectangular partition 12 having an opening in its top part, and a dividing piston 13 attached, with a fixed gap, to the bottom reflector 11. Said top reflector 10 is made to the shape of a cylinder whose base, as viewed from said partition, comprises an interior recess forming two symmetrical mirror surfaces tilted at an angle of 45° with respect to the cylinder axis. Said bottom reflector 11 is also made to the shape of a cylinder with its top base comprising two flat mirror faces symmetrically tilted at an angle of 45° with respect to the cylinder axis, the bottom base thereof being provided with a radial slot of a rectangular cross-section in a perpendicular direction to the plane of said partition. In so doing, the both reflectors are slightly smaller in diameter than a container inside diameter, thereby providing narrow gaps to force over and efficiently stir a medium to be studied. Said end openings 7 and 8 with pipe unions are communicated to each other through a stop valve 14 by means of a bypass tube 15.

In addition, said rectangular partition 12 is fixed with the aid of two grooves on the interior surface of the container in a perpendicular direction to the axis of the viewing ports 2.

An equilibrium bomb for studying a phase behavior of hydrocarbons operates as follows.

A high-pressure container 1 is filled with a medium to be studied, for example with a reservoir fluid or recombined sample, pressurized and heated to a required temperature by means of a thermo-static control system. Using a measuring press 9 that sets in motion a dividing piston 13, the contents of the container are reduced to specified thermo-dynamic conditions. Simultaneously, stirring of a medium to be studied occurs at the expense of its forcing over narrow gaps between reflectors 10, 11 and a container interior wall. Thereupon a phase state of the fluid is viewed visually through viewing ports 2 by setting particular thermo-baric modes.

### Mode 1. Determination of a Condensation Onset Pressure (Dew Point) of a Reservoir Gas-Condensate System

When a stop valve 14 of a bypass tube 15 is in closed position, a bottom light reflector 11 is set by means of a dividing piston 13 and a measuring press 9 at a level of the axis of viewing ports 2, thereupon this position is fixed by a pressure equalization in the space above and below the piston, when said stop valve 14 of said bypass tube 15 is in open position. Then, a rock pressure in the gas-condensate system is adjusted for a standard manometer connected to an equilibrium bomb through an opening with a pipe union. A luminous flux from a light source 3, after being passed over an inlet fiber-optic light conductor 4 and reflected from a bottom reflector 11, reaches a top reflector 10, passes through a circular opening made in a partition 12, returns to said bottom reflector 11, passes over an output fiber-optic light conductor 4 and reaches a viewing port 2. In the process for an isothermal depressurizing carried out by removing a part of a drawing-in working agent into said measuring press 9, at the moment of a "dew point" and occurrence of the first signs of fog (Pco), there is a sharp decrease in the brightness of a light beam that has passed a double distance between a bottom 11 and top 10 reflectors.

### Mode 2. Measurement of Micro Volumes of the Precipitated Hydrocarbon Condensate

Prior to measurements of volumes of a hydrocarbon condensate precipitated in depressurizing, first, when a stop valve 14 of a bypass tube 15 is in closed position, a through rectangular slot of a bottom reflector is set by means of a dividing piston and a measuring press 9 at a level of the axis of viewing ports. Thereupon, when a by-pass is in open position, a pressure in a single-phase gas-condensate system constituting a gaseous solution of hydrocarbon vapors, is lowered to a specified value below said Pc, and a gas - precipitated condensate equilibrium is set over a period of no less than 1.5-2 hours. When from a gaseous solution of hydrocarbons, a part of the condensate which could be precipitated at specified thermo-baric equilibrium conditions is completely removed, the by-pass is closed and, using the measuring press 9, a gas - condensate - drawing-in liquid phase boundary (a visible part of this boundary corresponds to the slot width) is sequentially set at a level of the slot and a central axis of a viewing port (having a special horizontal notch). Since the press allows for the measurement of only micro volumes of the precipitated hydrocarbon condensate through the said slot and narrow gap, a specified pressure is subjected no changes in the gas-condensate system. In so doing, precision measurements of micro volumes of a precipitated hydrocarbon condensate in accordance with the present invention are far superior to those described in the prior art.

### Mode 3. Visual Determination of a Saturation Pressure (Ps) of Reservoir Oil

In this case, when a stop valve 14 of a bypass tube 15 is in closed position, a dividing piston 13 and a measuring press 9 serve to bring a free space formed by a gap between a bottom light reflector 11 and said dividing piston 13 into coincidence with the axis of a viewing port 2, to enable a direct passage of a luminous flux from a light source 3 through a light oil to the opposite viewing port. Thereupon, when the top valve 14 of the bypass tube 15 is in open position, a reservoir pressure is set using the measuring press 9. In the process for an isothermal depressurizing (by removing a part of a drawing-in working agent into said measuring press), the moment of emergence and upward travel of bubbles of a liberated gas is observed.
In such a manner a saturation pressure (Ps) of light "volatile" stratified oil is set, and in case of darker oil, light source brightness is intensified.

The use of the present invention makes possible a significant expansion of the fields of applying the claimed equilibrium bomb through the provision of means for the study of a phase behavior of dark and light "volatile" stratified oils and also gas-condensate systems having a broad range of potential contents of C₅₊ liquid hydrocarbons, and an increase in precision measurements of micro volumes of a liquid phase precipitated there from when depressurizing.

Although the present invention has been described with reference to preferred embodiments, the invention is not limited to the details thereof and various changes and modifications obvious to one skilled in the art to which the invention pertains are deemed to be within the scope of the invention as further defined in the appended claims.

## Claims

1. An equilibrium bomb for studying a phase behavior of hydrocarbons comprising
- a temperature-controlled high-pressure cylindrical container (1) having face and side openings (5-8) with pipe unions,
- two viewing ports (2),
- a light source (3),
- top and bottom reflectors (10, 11) being rigidly connected to each other and disposed inside the container (1),
wherein
- the bomb is provided with a dividing piston (13) which is attached, with a fixed gap, to the bottom reflector (11),
- the bottom reflector (11) is made to the shape of a cylinder whose one base comprises two flat mirror faces symmetrically tilted at an angle of 45° with respect to the cylinder axis,
- the bomb is also provided with a bypass tube (15) connecting, through a stop valve (14), spaces above and below the piston (13) to each other,
- said reflectors (10, 11) are arranged with a gap relative to a container interior wall and connected to each other by means of a rectangular partition (12) being positioned along the axis of the container (1) and having an opening at a level of the top reflector (10),
- the top reflector (10) is made to the shape of a cylinder whose base, as viewed from the partition (12), comprises an interior recess forming two symmetrical mirror surfaces tilted at an angle of 45° with respect to the cylinder axis,
- the viewing ports (2) are provided at diametrically opposed locations on the lateral wall of the container (1) at a level of the light source (3), and
- the mirror faces of the top reflector (10) and of the bottom reflector (11) are arranged such that, when the bottom reflector is arranged at the level of the viewing ports, a luminous flux coming through a viewing port, from the light source (3) is reflected from the bottom reflector (11) to the top reflector (10), passed through the opening at a level of the top reflector (10), reflected from the top reflector (10) to the bottom reflector (11), and reflected from the bottom reflector (11) to the other viewing port (2).

2. A bomb as defined in claim 1, **characterized in that** the other base of the bottom reflector (11) is provided with a radial slot, and the radial slot of the bottom reflector (11) is arranged perpendicular to the partition (12), which is fixed with the aid of two grooves on the interior surface of the container (1) in a perpendicular direction to the axis of the viewing ports (2).

## Patentansprüche

1. Ein Gleichgesichtskolben zur Untersuchung eines Phasenverhaltens von Kohlenwasserstoffen aufweisend
- einen Temperatur-kontrollierten zylindrischen Hochdruck-Behälter (1), der eine Anlagefläche und Seitenöffnungen (5-8) mit Anschlussstutzen aufweist,
- zwei Beobachtungsfenster (2),
- eine Lichtquelle (3),
- obere und untere Reflektoren (10, 11), die fest aneinander gekoppelt und innerhalb des Behälters (1) angeordnet sind,
wobei
- der Kolben mit einem Trennkolben (13) versehen ist, der mit einem festen Spalt am unteren Reflektor (11) befestigt ist,
- der untere Reflektor (11) in Form eines Zylinders ausgebildet ist, dessen einer Boden zwei flache Spiegelflächen aufweist, die symmetrisch in einem Winkel von 45° in Bezug auf die Zylinderachse geneigt sind,
- der Kolben außerdem mit einem Bypass-Kanal (15) versehen ist, der durch ein Stopp-Ventil (14) Räume über und unterhalb des Kolbens (13) miteinander verbindet,
- die Reflektoren (10, 11) mit einem Spalt relativ zu einer Behälter-Innenwand angeordnet und miteinander mittels einer rechteckigen Trennwand (12) verbunden sind, die entlang der Achse des Behälters (1) positioniert ist und eine Öffnung auf einer Höhe des oberen Reflektors (10) aufweist,
- der obere Reflektor (10) in Form eines Zylinders ausgebildet ist, dessen einer Boden von der Membran (12) aus gesehen eine innere Aussparung aufweist, die zwei symmetrische Spiegelflächen ausbildet, die in einem Winkel von 45° in Bezug auf die Zylinderachse geneigt sind,
- die Beobachtungsfenster (2) an diametral gegenüberliegenden Stellen an der lateralen Wand des Behälters (1) auf einer Höhe der Lichtquelle (3) vorgesehen sind, und
- die Spiegelflächen des oberen Reflektors (10) und des unteren Reflektors (11) so angeordnet sind, dass, wenn der untere Reflektor auf der Höhe der Beobachtungsfenster angeordnet ist, ein durch ein Beobachtungsfenster von der Lichtquelle (3) kommender Lichtfluss vom unteren Reflektor (11) zum oberen Reflektor (10) reflektiert wird, durch die Öffnung auf einer Höhe des oberen Reflektors (10) hindurchtritt, vom oberen Reflektor (10) zum unteren Reflektor (11) reflektiert wird, und vom unteren Reflektor (11) zum anderen Beobachtungsfenster (2) reflektiert wird.

2. Ein Kolben wie in Anspruch 1 definiert, **dadurch gekennzeichnet, dass** der andere Boden des unteren Reflektors (11) mit einem radialen Schlitz versehen ist, und der radiale Schlitz des unteren Reflektors (11) rechtwinklig zur Trennwand (12) angeordnet ist, die mit Hilfe von zwei Nuten an der Innenfläche des Behälters (1) in einer rechtwinkligen Richtung zur Achse der Beobachtungsfenster (2) fixiert ist.

## Revendications

1. Une bombe d'équilibre pour étudier un comportement de phase des hydrocarbures comprenant
- une température contrôlée cylindrique conteneur de haute pression (1), qui a une surface d'appui et les ouvertures latérales (5-8) avec des adaptateurs,
- deux fenêtres d'observation (2),
- une source de lumière (3),
- réflecteurs haut et en bas (10, 11) étant rigidement reliés les uns aux autres et disposées à l'intérieur du conteneur (1), **caractérisé**
- la bombe est fourni d'un piston de division (13) qui est joint, avec un écart fixe, au réflecteur inférieur (11),
- le réflecteur inférieur (11) est établie à la forme d'un cylindre dont une base comporte deux faces planes miroir symétriquement incliné à un angle de 45° par rapport à l'axe du cylindre,
- la bombe est également fourni avec un tube de dérivation (15) reliant, à travers une vanne d'arrêt (14), des espaces ci-dessus et en dessous du piston (13) les uns aux autre,
- dit réflecteurs (10, 11) sont disposées avec un écart par rapport à un mur intérieur du conteneur et reliés les uns aux autres au moyen d'une partition rectangulaire (12) étant positionné dans l'axe du conteneur (1) et présentant une ouverture à un niveau du réflecteur supérieur (10),
- le réflecteur supérieur (10) est faite de la forme d'un cylindre dont la base, vu de la partition (12), comporte un évidement intérieur formant deux miroirs surfaces symétriques inclinés à un angle de 45° par rapport à l'axe du cylindre,
- les fenêtres d'observation (2) sont fournis à des endroits diamétralement opposés sur la paroi latérale du conteneur (1) à une hauteur de source lumineuse (3), et
- le miroir des surfaces du réflecteur supérieur (10) et réflecteur inférieur (11) sont disposés de manière que lorsque le réflecteur inférieur est organisée à la hauteur de les fenêtres d'observation, une rivière de lumière qui passe par une fenêtre d'observation de la source lumineuse (3) est réfléchi par le réflecteur inférieur (11) à réflecteur supérieur (10), passe par l'ouverture à une élévation du réflecteur supérieur (10), est réfléchi par le réflecteur supérieur (10) à réflecteur inférieur (11), et est réfléchi par le réflecteur inférieur (11) à une autre fenêtre d'observation (2).

2. Une bombe tel que défini dans la revendication 1, **caractérisé en ce que** l'autre base du réflecteur inférieur (11) est muni d'une fente radiale, et la fente radiale du réflecteur inférieur (11) est disposé perpendiculairement à la partition (12), qui est fixé à l'aide de deux rainures sur la surface intérieure du conteneur (1) dans une direction perpendiculaire à l'axe de la fenêtre d'observation (2).
